# EUROPEAN PATENT APPLICATION

(11) **EP 3 225 220 A1**
(43) Date of publication of application: **04.10.2017**
(21) Application number: 16002039.2
(22) Date of filing: 20.09.2016
(51) Int. Cl.: A61F 5/01, A61Q 3/00, A61K 8/02, A43B 3/00, A61K 9/70

(54) **HEEL CARING FILM**

(30) Priority: 31.03.2016 TW 16105204
(71) Applicant: Top Yield T.J. Enterprise Co., Ltd, Xinfeng Township (Hsinchu County) (TW)
(72) Inventor: CHAN, Jui-Mei, Xinfeng Township (Hsinchu County) (TW)
(74) Representative: Zeitler Volpert Kandlbinder Patent- und Rechtsanwälte Partnerschaft mbB

(57) **Abstract**

The present invention discloses a heel caring film. The heel caring film is a bag-shaped structure formed by enclosing with two pieces of water-proofing substrate to be worn on the heel of a user. A corner of each water-proofing substrate is sealed, forming a closure edge corresponding to a rear side and bottom side of the heel. The other corner of each water-proofing substrate opposite to the closure edge is extended respectively to form a joining part which encloses the foot dorsum of the user. In addition, an interior side of the water-proofing substrate is coated with a layer of water-retention material corresponding to the heel of the user. Accordingly, a heel caring film which can be used to care the heel of the user and can be worn on the heel of the user more comfortably as well as conveniently is achieved.

## Description

### BACKGROUND OF THE INVENTION

### a) Field of the Invention

The present invention relates to a body caring product, and more particularly to a heel caring film which can be used to care the heel of a user and can be worn on the heel of the user more comfortably as well as conveniently.

### b) Description of the Prior Art

In terms of the skin physiology, the corneum is the outermost layer on human skin, responsible for protecting internal tissues and organs. In other words, on the part of a human body which moves more often, the corneum will be thicken constantly due to the friction on the skin, so as to protect the skin. In particular, as the heels support the weight of the whole body and need to move often, the foot skin can be aging, roughening or dry cracking more easily by time, environment or wearing high heels, as compared to other parts.

The cornified cells in the corneum are basically old and wasted, as this is a very important process in the physiological metabolism of the cornified cells of the skin. Even without an external force to remove the skin corneum, there is still a certain metabolism schedule for the skin corneum. In principle, if we can keep the skin clean and maintain the moisturizing function of the corneum regularly, the corneum can fall out for replacement by itself to keep renewing.

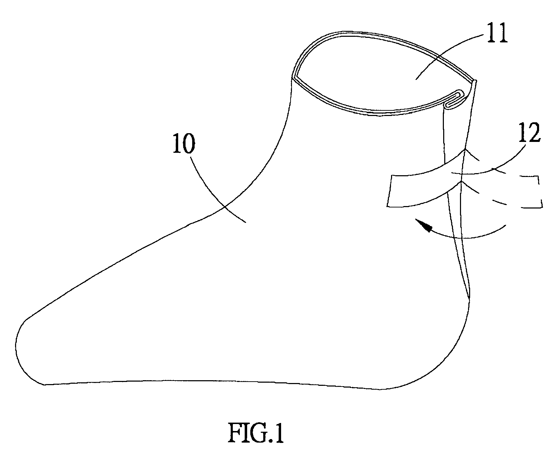

However, for a female who needs to wear high heels often, it is very eager for her to avoid that the skin is too rough and not smooth as the heel corneum is too thick. Therefore, there is a foot film on the markets for a consumer to care the heel skin by oneself. As shown in FIG. 1, a conventional foot film includes a bag 10 for inserting the sole of the user. The bag 10 is provided with an opening 11 corresponding to an upper side of the user's ankle, and a fixing element 12 which keeps the opening 11 at the bonded state.

Nevertheless, as the growth rate is different between the corneum of the heels and the corneum of the toes, the timing for removing the corneum will be different too. When the abovementioned conventional foot film is in use, the heels cannot be cared and the whole sole has to be inserted into the bag 10, such that the heels can be moisturized by a caring agent in the bag 10 for a long time, which results in that the user may feel that the toes are sultry and uncomfortable or the toes may even get infected as the toes are soaked in the caring agent at a same time.

Furthermore, when the abovementioned conventional foot film is in use, the whole sole is enclosed, thus the user can hardly walk around while wearing the foot film. In addition, an awkward thing will usually happen that the sole is too big to be sheathed into the foot film or the sole is too small that the foot film cannot enclose the foot tightly to form a gap.

### SUMMARY OF THE INVENTION

Accordingly, the primary object of the present invention is to provide a heel caring film that can be used to care the heel of the user, and can be worn on the heel of the user more comfortably as well as conveniently.

The heel caring film of the present invention is a bag-shaped structure formed by enclosing with two pieces of water-proofing substrate to be worn on the heel of the user. A corner of each water-proofing substrate is sealed, forming a closure edge corresponding to a rear side and a bottom side of the heel. The other corner of each water-proofing substrate opposite to the closure edge is extended respectively to form a joining part which encloses the foot dorsum of the user. In addition, an interior side of the water-proofing substrate is coated with a layer of water-retention material corresponding to the heel of the user.

In principle, when the heel caring film of the present invention is in use, the user encloses the joining parts of the two water-proofing substrates on two sides of the sole toward the foot dorsum in a crisscross way, and then fixes the joining parts of the two water-proofing substrates by adhering in order to care the heel of the user. In particular, as the toes of the user are exposed outside when the user is wearing the whole heel caring film, he or she will feel more comfortable and the toes will not get infected by being soaked in the caring agent too long. In addition, when necessary, the user can walk freely by tiptoeing, which further manifests the comfortableness and the convenience in wearing the whole heel caring film.

In accordance with the abovementioned structural features, for the said heel caring film, an interior side of each water-proofing substrate is provided with a lower water-stopping strip which is extended along a lower edge of the joining part to connect with the closure edge.

In accordance with the abovementioned structural features, for the said heel caring film, an interior side of each water-proofing substrate is provided with a lower water-stopping strip which is extended along a lower edge of the joining part to connect with the closure edge, and an interior side of each water-proofing substrate is provided with an upper water-stopping strip which is extended along an upper edge of the joining part to connect with the closure edge.

In accordance with the abovementioned structural features, for the said heel caring film, an outer periphery of each water-proofing substrate is sealed correspondingly with a sealing edge which is connected with the closure edge.

In accordance with the abovementioned structural features, for the said heel caring film, an outer periphery of each water-proofing substrate is sealed correspondingly with a sealing edge which is connected with the closure edge, and at least one water-proofing substrate is marked with a trim line which is disposed along the sealing edge.

In accordance with the abovementioned structural features, for the said heel caring film, an outer periphery of each water-proofing substrate is sealed correspondingly with a sealing edge which is connected with the closure edge, and an interior side of each water-proofing substrate is provided with a lower water-stopping strip which is extended along a lower edge of the joining part to connect with the closure edge.

In accordance with the abovementioned structural features, for the said heel caring film, an outer periphery of each water-proofing substrate is sealed correspondingly with a sealing edge which is connected with the closure edge, an interior side of each water-proofing substrate is provided with a lower water-stopping strip which is extended along a lower edge of the joining part to connect with the closure edge, and at least one water-proofing substrate is marked with a trim line which is disposed along the sealing edge.

In accordance with the abovementioned structural features, for the said heel caring film, an outer periphery of each water-proofing substrate is sealed correspondingly with a sealing edge which is connected with the closure edge, an interior side of each water-proofing substrate is provided with a lower water-stopping strip which is extended along a lower edge of the joining part to connect with the closure edge, and an interior side of each water-proofing substrate is provided with an upper water-stopping strip which is extended along an upper edge of the joining part to connect with the closure edge.

In accordance with the abovementioned structural features, for the said heel caring film, an outer periphery of each water-proofing substrate is sealed correspondingly with a sealing edge which is connected with the closure edge, an interior side of each water-proofing substrate is provided with a lower water-stopping strip which is extended along a lower edge of the joining part to connect with the closure edge, an interior side of each water-proofing substrate is provided with an upper water-stopping strip which is extended along an upper edge of the joining part to connect with the closure edge, and at least one water-proofing substrate is marked with a trim line which is disposed along the sealing edge.

In accordance with the abovementioned structural features, the said heel caring film is loaded with a caring agent, and an outer periphery of each water-proofing substrate is sealed correspondingly with a sealing edge which is connected with the closure edge.

In accordance with the abovementioned structural features, the said heel caring film is loaded with a caring agent, an outer periphery of each water-proofing substrate is sealed correspondingly with a sealing edge which is connected with the closure edge, and at least one water-proofing substrate is marked with a trim line which is disposed along the sealing edge.

In accordance with the abovementioned structural features, the said heel caring film is loaded with a caring agent, an outer periphery of each water-proofing substrate is sealed correspondingly with a sealing edge which is connected with the closure edge, and an interior side of each water-proofing substrate is provided with a lower water-stopping strip which is extended along a lower edge of the joining part to connect with the closure edge.

In accordance with the abovementioned structural features, the said heel caring film is loaded with a caring agent, an outer periphery of each water-proofing substrate is sealed correspondingly with a sealing edge which is connected with the closure edge, an interior side of each water-proofing substrate is provided with a lower water-stopping strip which is extended along a lower edge of the joining part to connect with the closure edge, and at least one water-proofing substrate is marked with a trim line which is disposed along the sealing edge.

In accordance with the abovementioned structural features, the said heel caring film is loaded with a caring agent, an outer periphery of each water-proofing substrate is sealed correspondingly with a sealing edge which is connected with the closure edge, an interior side of each water-proofing substrate is provided with a lower water-stopping strip which is extended along a lower edge of the joining part to connect with the closure edge, and an interior side of each water-proofing substrate is provided with an upper water-stopping strip which is extended along an upper edge of the joining part to connect with the closure edge.

In accordance with the abovementioned structural features, the said heel caring film is loaded with a caring agent, an outer periphery of each water-proofing substrate is sealed correspondingly with a sealing edge which is connected with the closure edge, an interior side of each water-proofing substrate is provided with a lower water-stopping strip which is extended along a lower edge of the joining part to connect with the closure edge, an interior side of each water-proofing substrate is provided with an upper water-stopping strip which is extended along an upper edge of the joining part to connect with the closure edge, and at least one water-proofing substrate is marked with a trim line which is disposed along the sealing edge.

For the said heel caring film, a layer of self-adhering glue is disposed near a tail end on an interior side of the joining part of at least one water-proofing substrate, and a surface on the layer of the self-adhering glue is covered with a release material which can be torn apart.

An outer profile along which the lower edge on the tail end of the joining part of the said each water-proofing substrate is extended to the closure edge is in a shape of an arc concaving into the heel caring film.

For the said heel caring film, a layer of self-adhering glue is disposed near a tail end on an interior side of the joining part of at least one water-proofing substrate, a surface on the layer of the self-adhering glue is covered with a release material which can be torn apart, and an outer profile along which the lower edge on the tail end of the joining part of each water-proofing substrate is extended to the closure edge is in a shape of an arc concaving into the heel caring film.

The said water-proofing substrate is made of non-woven fabric.

The heel caring agent disclosed by the present invention can be used to care the heel of the user; whereas, as the toes of the user are exposed outside, the user can wear the heel caring film more comfortably and the toes will not get infected by being soaked in the caring agent too long. In addition, when necessary, the user can walk freely by tiptoeing, which further manifests the comfortableness and convenience in wearing the whole heel caring film. In particular, as the outer profile along which the lower edge on the tail end of the joining part of the water-proofing substrate is extended to the closure edge is in a shape of an arc concaving into the heel caring agent, the whole heel caring film can be attached better with the arch and the foot dorsum of the user, and the tail end on the closure edge can be even lifted up, which prevents the caring agent from leaking out in a more aggressive and reliable means.

To enable a further understanding of the said objectives and the technological methods of the invention herein, the brief description of the drawings below is followed by the detailed description of the preferred embodiments.

### BRIEF DESCRIPTION OF THE DRAWINGS

FIG. 1 shows a schematic view of a usage state of a conventional foot film.
FIG. 2 shows a structural schematic view of a preferred embodiment of a heel caring film, according to the present invention.
FIG. 3 shows a structural schematic view of the heel caring film of the present invention after a sealing edge is cut open.
FIG. 4 shows another structural schematic view of the heel caring film of the present invention after the sealing edge is cut open.
FIG. 5 shows a schematic view of a state when the heel caring film of the present invention is sheathed on the heel of a user.
FIG. 6 shows a schematic view of another way of fixing when the user is wearing the heel caring film of the present invention.

### DETAILED DESCRIPTION OF THE PREFERRED EMBODIMENTS

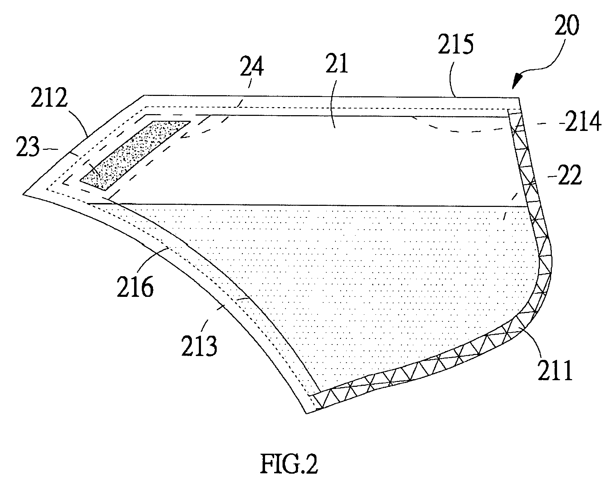

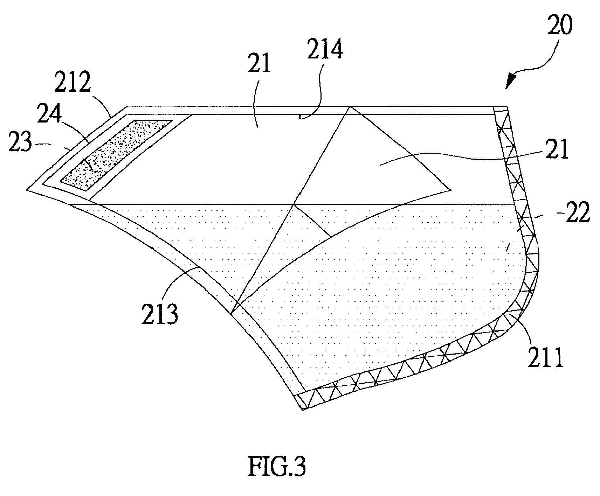

The present invention discloses primarily a heel caring film which can be used to care the heel of the user and can be worn on the heel more comfortably as well as conveniently. As shown in FIG. 2 and FIG. 3, the heel caring film 20 of the present invention is a bag-shaped structure formed by enclosing with two pieces of water-proofing substrate 21 to be worn on the heel of the user. A corner of each water-proofing substrate 21 is sealed to form a closure edge 211 corresponding to a rear side and a bottom side of the heel of the user. The other corner of each water-proofing substrate 21 opposite to the closure edge 211 is extended respectively to form a joining part 212 which encloses the foot dorsum of the user.

An interior side of each water-proofing substrate 21 is coated with a layer of water-retention material 22 corresponding to the heel of the user. Upon implementation, the said water-retention material 22 can be made of non-woven fabric or other material which can adsorb water. According to the abovementioned structural features, the heel caring film of the present invention can be manifested as a product allowing the user to add a caring agent by oneself or be manifested as a product which is pre-loaded with a caring agent.

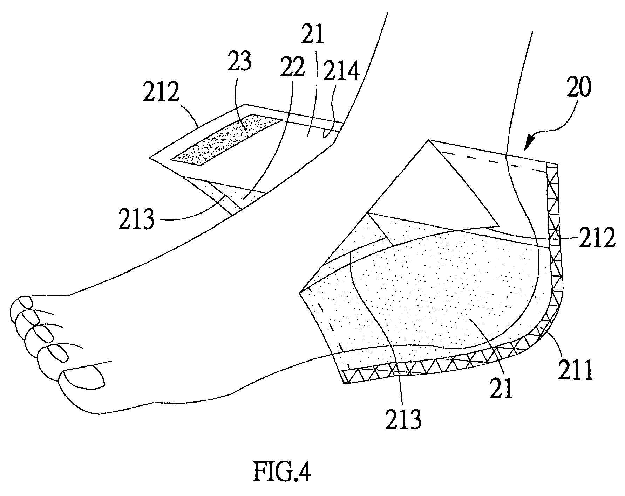

In principle, when the heel caring film of the present invention is in use, as shown in FIG. 4, under a condition that the water-retention material 22 has already adsorbed a caring agent, the whole heel caring film 20 is sheathed on the heel of the user by expanding outward the joining parts 212 of the two water-proofing substrates 21.

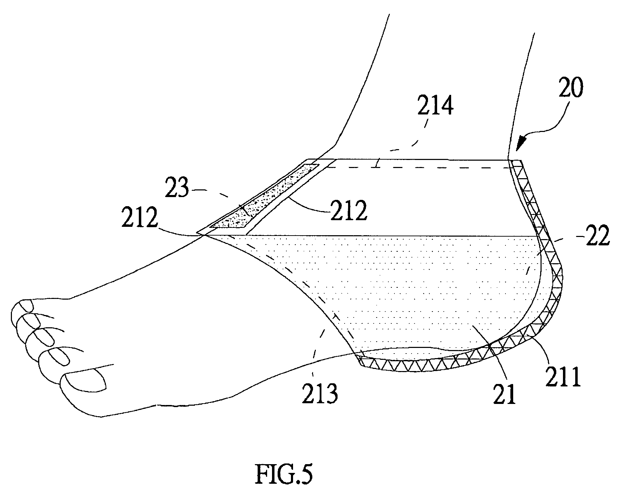

Next, by enclosing the joining parts 212 of the two water-proofing substrates 21 on two sides of the sole toward the foot dorsum in a crisscross way, the whole heel caring film 20 can be worn on the heel of the user; and as shown in FIG. 5, the joining parts 212 of the two water-proofing substrates 21 are fixed by adhering, so as to care the heel of the user.

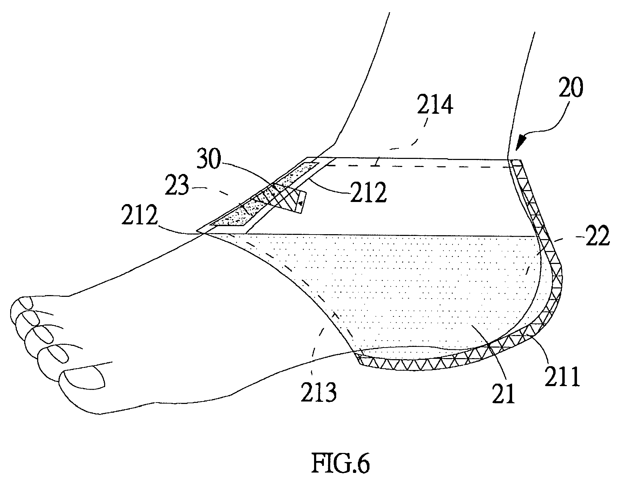

In practically using the heel caring film of the present invention, the joining parts 212 of the two water-proofing substrates 21 can be also fixed by a tape 30, as shown in FIG. 6. As the toes of the user are exposed outside when the user is wearing the heel caring film, he or she will feel more comfortable and the toes will not get infected by being soaked in the caring agent too long. In addition, when necessary, the user can walk freely by tiptoeing, which further manifests the comfortableness and the convenience in wearing the whole heel caring film.

Referring to FIG. 2 and FIG. 3 at a same time, upon implementation, an interior side of each water-proofing substrate 21 can be further provided with a lower water-stopping strip 213 which is extended along a lower edge of the joining part 212 to connect with the closure edge 211. By this lower water-stopping strip 213, a better contact area between the heel caring film 20 and the skin of the user can be formed to prevent the caring agent from leaking out.

In the present embodiment, for the whole heel caring film 20, an interior side of each water-proofing substrate 21 is provided with a lower water-stopping strip 213 which is extended along a lower edge of the joining part 212 to connect with the closure edge 211, and an interior side of each water-proofing substrate 21 is also provided with an upper water-stopping strip 214 which is extended along an upper edge of the joining part 212 to connect with the closure edge 211.

Moreover, whether the heel caring film 20 of the present invention is loaded with a caring agent, an outer periphery of each water-proofing substrate 21 is further sealed correspondingly with a sealing edge 215 which is connected with the closure edge 211. In addition, under the structure configuration that the outer periphery of each water-proofing substrate 21 is sealed correspondingly with the sealing edge 215 which is connected with the closure edge 211, at least one water-proofing substrate 21 is marked with a trim line 216 which is disposed along the sealing edge 215, facilitating the user to cut open the sealing edge 215 outside the closure edge 211 along the trim line 216 for use (as shown in FIG. 4).

In the abovementioned embodiment as shown in FIG. 2 and FIG. 3, for the whole heel caring film 20, an outer periphery of each water-proofing substrate 21 is sealed correspondingly with a sealing edge 215 which is connected with the closure edge 211, an interior side of each water-proofing substrate 21 is provided with a lower water-stopping strip 213 which is extended along a lower edge of the joining part 212 to connect with the closure edge 211, an interior side of each water-proofing substrate 21 is provided with an upper water-stopping strip 214 which is extended along an upper edge of the joining part 212 to connect with the closure edge 211, and at least one water-proofing substrate 21 is marked with a trim line 216 which is disposed along the sealing edge 215.

When implementing the heel caring film of the present invention, the product can be also manifested as that the outer periphery of each water-proofing substrate is sealed correspondingly with the sealing edge which is connected with the closure edge, and the interior side of each water-proofing substrate is provided with the lower water-stopping strip which is extended along the lower edge of the joining part to connect with the closure edge.

Or the product can be manifested as that the outer periphery of each water-proofing substrate is sealed correspondingly with the sealing edge which is connected with the closure edge, the interior side of each water-proofing substrate is provided with the lower water-stopping strip which is extended along the lower edge of the joining part to connect with the closure edge, and at least one water-proofing substrate is marked with the trim line which is disposed along the sealing edge.

Or the product can be manifested as that the outer periphery of each water-proofing substrate is sealed correspondingly with the sealing edge which is connected with the closure edge, the interior side of each water-proofing substrate is provided with the lower water-stopping strip which is extended along the lower edge of the joining part to connect with the closure edge, and the interior side of each water-proofing substrate is provided with the upper water-stopping strip which is extended along the upper edge of the joining part to connect with the closure edge.

It is naturally that when the heel caring film 20 of the present invention is manifested as the product which is loaded with a caring agent (without a drawing), it is preferred that the whole heel caring film 20 is as shown in the drawings that the outer periphery of each water-proofing substrate 21 is sealed correspondingly with the sealing edge 215 which is connected with the closure edge 211, along with the lower water-stopping strip 213, the upper water-stopping strip 214, the trim line 216 or the combination thereof optionally.

Moreover, under the abovementioned structural configurations that may be implemented, the whole heel caring film 20 of the present invention can be shown in FIG. 3 that a layer of self-adhering glue 23 is disposed near a tail end on the interior side of the joining part 212 of at least one water-proofing substrate 21, and a surface on the layer of the self-adhering glue 23 is covered with a release material 24 which can be torn apart, thereby facilitating a user to fix directly the joining part 212 of the water-proofing substrate 21 by the layer of the self-adhering glue 23, after tearing apart the release material 24.

In addition, the outer profile along which the lower edge on the tail end of the joining part 212 of the said water-proofing substrate 21 is extended to the closure edge 211 is shown in FIG. 3, and it is preferred that the outer profile is in a shape of an arc concaving into the heel caring film 20 as shown in FIG. 5. The whole heel caring film 20 can be attached better with the arch and the foot dorsum of the user, and the tail end on the closure edge 211 can be even lifted up, which is able to prevent the caring agent from leaking out in a more aggressive and reliable means.

It is naturally that for the whole heel caring film 20, the layer of the self-adhering glue 23 is disposed near the tail end on the interior side of the joining part 212 of at least one water-proofing substrate 21, and the surface on the layer of the self-adhering glue 23 is covered with the release material 24 which can be torn apart. In addition, it is preferred to manifest the structural configuration that the outer profile, along which the lower edge on the tail end of the joining part 212 of the water-proofing substrate 21 is extended to the closure edge 211, is in the shape of an arc concaving into the heel caring film 20.

As compared to the prior art, the heel caring film of the present invention can be used to care the heel of the user, and the toes of the user are exposed outside; therefore, the user will feel more comfortable in wearing the heel caring film and the toes will not get infected by being soaked in the caring agent too long. In addition, when necessary, the user can walk freely by tiptoeing, which further manifests the comfortableness and the convenience in wearing the heel caring film. In particular, as the outer profile along which the lower edge on the tail end of the joining part of the water-proofing substrate is extended to the closure edge is in the shape of an arc concaving into the heel caring film, the whole heel caring film can be attached better with the arch and the foot dorsum of the user and the tail end on the closure edge can be even lifted up, which prevents the caring agent from leaking out in a more aggressive and reliable means.

It is of course to be understood that the embodiments described herein is merely illustrative of the principles of the invention and that a wide variety of modifications thereto may be effected by persons skilled in the art without departing from the spirit and scope of the invention as set forth in the following claims.

## Claims

1. A heel caring film (20) being a bag-shaped structure formed by enclosing with two pieces of water-proofing substrate (21) to be worn on the heel of a user, with that a corner of each water-proofing substrate (21) is sealed to form a closure edge (211) corresponding to a rear side and a bottom side of the heel, the other corner of each water-proofing substrate (21) opposite to the closure edge (211) is extended respectively to form a joining part (212) which encloses the foot dorsum of the user, and an interior side of the water-proofing substrate (21) is coated with a layer of water-retention material (22) corresponding to the heel of the user.

2. The heel caring film (20) according to claim 1, wherein an outer periphery of each water-proofing substrate (21) is sealed correspondingly with a sealing edge (215) which is connected with the closure edge (211), an interior side of each water-proofing substrate (21) is provided with a lower water-stopping strip (213) which is extended along a lower edge of the joining part (212) to connect with the closure edge (211), and one water-proofing substrate (21) is marked with a trim line (216) which is disposed along the sealing edge (215).

3. The heel caring film (20) according to claim 1, wherein an outer periphery of each water-proofing substrate (21) is sealed correspondingly with a sealing edge (215) which is connected with the closure edge (211), an interior side of each water-proofing substrate (21) is provided with a lower water-stopping strip (213) which is extended along a lower edge of the joining part (212) to connect with the closure edge (211), and an interior side of each water-proofing substrate (21) is provided with an upper water-stopping strip (214) which is extended along an upper edge of the joining part (212) to connect with the closure edge (211).

4. The heel caring film (20) according to claim 1, wherein an outer periphery of each water-proofing substrate (21) is sealed correspondingly with a sealing edge (215) which is connected with the closure edge (211), an interior side of each water-proofing substrate (21) is provided with a lower water-stopping strip (213) which is extended along a lower edge of the joining part (212) to connect with the closure edge (211), an interior side of each water-proofing substrate (21) is provided with an upper water-stopping strip (214) which is extended along an upper edge of the joining part (212) to connect with the closure edge (211), and one water-proofing substrate (21) is marked with a trim line (216) which is disposed along the sealing edge (215).

5. The heel caring film (20) according to claim 1, wherein the heel caring film (20) is loaded with a caring agent, an outer periphery of each water-proofing substrate (21) is sealed correspondingly with a sealing edge (215) which is connected with the closure edge (211), an interior side of each water-proofing substrate (21) is provided with a lower water-stopping strip (213) which is extended along a lower edge of the joining part (212) to connect with the closure edge (211), and one water-proofing substrate (21) is marked with a trim line (216) which is disposed along the sealing edge (215).

6. The heel caring film (20) according to claim 1, wherein the heel caring film (20) is loaded with a caring agent, an outer periphery of each water-proofing substrate (21) is sealed correspondingly with a sealing edge (215) which is connected with the closure edge (211), an interior side of each water-proofing substrate (21) is provided with a lower water-stopping strip (213) which is extended along a lower edge of the joining part (212) to connect with the closure edge (211), and an interior side of each water-proofing substrate (21) is provided with an upper water-stopping strip (214) which is extended along an upper edge of the joining part (212) to connect with the closure edge (211).

7. The heel caring film (20) according to claim 1, wherein the heel caring film (20) is loaded with a caring agent, an outer periphery of each water-proofing substrate (21) is sealed correspondingly with a sealing edge (215) which is connected with the closure edge (211), an interior side of each water-proofing substrate (21) is provided with a lower water-stopping strip (213) which is extended along a lower edge of the joining part (212) to connect with the closure edge (211), an interior side of each water-proofing substrate (21) is provided with an upper water-stopping strip (214) which is extended along an upper edge of the joining part (212) to connect with the closure edge (211), and one water-proofing substrate (21) is marked with a trim line (216) which is disposed along the sealing edge (215).

8. The heel caring film (20) according to claim 1, 2, 3, 4, 5, 6 or 7, wherein a layer of self-adhering glue (23) is disposed near a tail end on an interior side of the joining part (212) of one water-proofing substrate (21), and a surface on the layer of the self-adhering glue (23) is covered with a release material (24) which is to be torn apart.

9. The heel caring film (20) according to claim 1, 2, 3, 4, 5, 6 or 7, wherein an outer profile along which the lower edge on the tail end of the joining part (212) of the water-proofing substrate (21) is extended to the closure edge (211) is in a shape of an arc concaving into the heel caring film (20).

10. The heel caring film (20) according to claim 1, 2, 3, 4, 5, 6 or 7, wherein a layer of self-adhering glue (23) is disposed near a tail end on an interior side of the joining part (212) of one water-proofing substrate (21), a surface on the layer of the self-adhering glue (23) is covered with a release material (24) which is to be torn apart, and an outer profile along which the lower edge on the tail end of the joining part (212) of the water-proofing substrate (21) is extended to the closure edge (211) is in a shape of an arc concaving into the heel caring film (20).

11. The heel caring film (20) according to claim 1, 2, 3, 4, 5, 6 or 7, wherein the water-retention material (22) is made of non-woven fabric.
